Europäisches Patentamt

European Patent Office

Office européen des brevets

(19)

(11) Veröffentlichungsnummer: **0 373 469 B1**

(12)

# EUROPÄISCHE PATENTSCHRIFT

(45) Veröffentlichungstag der Patentschrift: **08.09.93**

(51) Int. Cl.5: **A61K 7/16**, B65D 35/24

(21) Anmeldenummer: **89122341.4**

(22) Anmeldetag: **04.12.89**

Verbunden mit 90900130.7/0447434
(europäische
Anmeldenummer/Veröffentlichungsnummer)
durch Entscheidung vom 16.12.91.

(54) **Zahnpaste.**

(30) Priorität: **12.12.88 DE 3841775**

(43) Veröffentlichungstag der Anmeldung:
**20.06.90 Patentblatt 90/25**

(45) Bekanntmachung des Hinweises auf die
Patenterteilung:
**08.09.93 Patentblatt 93/36**

(84) Benannte Vertragsstaaten:
**AT BE CH DE ES FR GB GR IT LI NL SE**

(56) Entgegenhaltungen:
**EP-A- 0 076 563**
**US-A- 3 240 393**

(73) Patentinhaber: **Henkel Kommanditgesellschaft
auf Aktien**

**D-40191 Düsseldorf(DE)**

(72) Erfinder: **Kornettka, Norbert
Kalkarer Strasse 18
D-4000 Düsseldorf 11(DE)**
Erfinder: **Förg, Franz, Dr.
Rotdornweg 10
D-4018 Langenfeld(DE)**
Erfinder: **Stöffler, Albert
Nikolausstrasse 39
D-4000 Düsseldorf 13(DE)**
Erfinder: **Fiedler, Jürgen
Hombergen 15
D-4054 Nettetal 1(DE)**

**Beschreibung**

Gegenstand der Erfindung sind Zahnpasten in Form eines mehrfarbigen, gestreiften Stranges sowie eine Vorrichtung zum Ausbringen des Stranges aus einem Behälter.

Bei Produkten auf dem Gebiet der Körperpflege und Kosmetik kommt neben den entsprechenden Inhaltsstoffen auch einer ansprechenden Darreichungsform eine ständig wachsende Bedeutung zu. Dabei können neben der reinen Ästhetik, die bei der Auswahl von Farbe, Verpackung und häufig auch der Konsistenz des Produktes eine große Rolle spielt, auch praktische Gesichtspunkte auftreten. So läßt sich beispielsweise die Freude von Kindern an Farben und Formen darauf ausrichten, daß sich die Kinder gleichsam spielerisch mit notwendigen Vorgängen zu Reinigung und Pflege des Körpers vertraut machen. Eine Möglichkeit, Kinder zu einer regelmäßigen, intensiven Pflege des Gebisses zu erziehen, wird in der Bereitstellung einer - möglichst mehrfarbig - gefärbten, durch eine ansprechende Form des Stranges ausgezeichneten und gegebenenfalls mit einem angenehmen Aroma versehenen Zahnpaste gesehen.

Zahnpasten mit eingelagerten Kernen oder Streifen sind bekannt. So beschreibt die deutsche Offenlegungsschrift 19 37 001 Zahnpasten, bei denen in einen gelartigen Trägerstrang Streifen oder Kerne einer weiteren pastenförmigen Masse mit hoher Reinigungswirkung eingelagert sind. Der umgekehrte Fall, bei dem in einen undurchsichtigen Zahnpaste-Trägerstrang mit hoher Reinigungswirkung durchsichtige Streifen eingelagert werden, ist der europäischen Patentanmeldung 76 563 zu entnehmen. In beiden Schriften wird darauf hingewiesen, daß Trägerstrangmasse und Streifenmasse möglichst gleiche rheologische Eigenschaften aufweisen sollten. Weitere Beispiele für gestreifte Zahnpasten finden sich in den deutschen Offenlegungsschriften 16 17 907 und 24 54 136.

Gestreifte Zahnpastenstränge werden üblicherweise dadurch erzeugt, daß beim Druck auf den Vorratsbehälter gleichzeitig Trägerstrangmasse und Streifenmasse abgegeben werden. Das gewünschte Erscheinungsbild des Zahnpastenstranges wird durch entsprechende Konstruktion der Auslaßöffnung des Vorratsbehälters bestimmt. Informationen zum Füllen entsprechender Zahnpasten-Tuben und zur Gestaltung der Auslaßöffnung dieser Tuben finden sich beispielsweise in der deutschen Patentschrift 11 85 981 beziehungsweise der britischen Patentschrift 813 514.

Die bekannten Streifenzahnpasten weisen einen etwa kreisförmigen, meist wenig stabilen Strangquerschnitt auf. Auch bildet sich zwischen dem Trägerstrang und den Streifen häufig ein diffuser Übergangsbereich aus.

Dieser Übergangsbereich sollte zur Steigerung des ästhetischen Erscheinungsbildes des Stranges eliminiert werden. Des weiteren besteht das Bedürfnis nach Zahnpasten-Strängen mit polygonem Querschnitt, die auf Zahnbürsten einen verbesserten Halt haben. Dies ist besonders von Interesse, wenn die Zahnbürsten von Kinderhand geführt werden. In den Kanten eines derartigen Zahnpasten-Stranges können sich nunmehr die andersfarbigen Streifen befinden.

Hierzu sollen der Tragerstrangmasse und die Streifenmasse derart aufeinander abgestimmt sein, daß sie synchron und gleichmäßig aus einem Behälter auszubringen sind, wobei die Streifenmasse insbesondere die Eckkanten eines mehreckigen Zahnpastenstranges ausbilden soll.

Weiterhin soll eine Vorrichtung geschaffen werden, die das synchrone und gleichmäßige Ausbringen der beiden aufeinander abgestimmten Zahnpasten-Phasen ermöglicht.

Es wurde nun überraschenderweise gefunden, daß die Aufgabe dadurch gelöst werden kann, daß man Trägerstrangmassen und Streifenmassen so formuliert, daß sie sich in bestimmten rheologischen Parametern in definierter Weise voneinander unterscheiden.

Gegenstand der Erfindung ist somit eine Zahnpaste zum Ausbringen aus einem Behälter in Form eines mehrfarbigen, gestreiften Stranges, bestehend aus einer Trägerstrangmasse sowie mindestens einer, in der Farbe von der Trägerstrangmasse verschiedenen streifenmasse, dadurch gekennzeichnet, daß das Verhältnis der Fließgrenzen von Trägerstrangmasse und Streifenmasse 1,10:1 bis 1,20:1 beträgt.

Zahnpasten zeigen üblicherweise das rheologische Verhalten nichtnewtonscher Flüssigkeiten. Beim Anlegen einer Schubspannung, die unterhalb eines als Fließgrenze bezeichneten Wertes liegt, verhält sich die Substanz wie ein Festkörper. Ein Fließen der Substanz wird erst beim Anlegen von Schubspannungen beobachtet, die größer als dieser Grenzwert sind. Der funktionale Zusammenhang zwischen angelegter Schubspannung und beobachteter Schergeschwindigkeit oberhalb der Fließgrenze kann mit Hilfe semiempirischer Zustandsgleichungen, wie beispielsweise der Bingham-Gleichung oder der Casson-Gleichung beschrieben werden. In den meisten Fällen wird das rheologische Verhalten von Zahnpasten recht gut durch die Casson-Gleichung

$$D = (\sqrt{\tau} - \sqrt{f_c})^2 / \eta$$

EP 0 373 469 B1

beschrieben, wobei die einzelnen Größen folgende Bedeutung haben:

D Schergeschwindigkeit

$\tau$ Schubspannung

$f_c$ Fließgrenze (nach Casson)

$\eta$ dynamische Viskosität.

Nähere Einzelheiten zu diesen rheologischen Beziehungen sind der einschlägigen Fachliteratur, beispielsweise den Veröffentlichungen von R.R. Zangger in CZ-Chemie-Technik, 3. Jahrgang, S. 283-286 (1974), G. Simon in Chemiker-Zeitung, 100. Jahrgang, S. 25-34 (1976) und B. Idson in Cosmetics and Toiletries, Vol 93, S. 23-30 (1978), zu entnehmen.

Die rheologischen Eigenschaften der Zahnpasten lassen sich mit den handelsüblichen Viskosimetern, beispielsweise den Rotationsviskosimetern der Fa. Haake, bestimmen. In einer Grafik, in der die jeweilige Schergeschwindigkeit als Funktion der Schubspannung aufgetragen wird, läßt sich die Fließgrenze, gegebenenfalls durch Extrapolation der Meßkurve auf D = 0, in für den Fachmann bekannter Weise ablesen. Zeigt die Zahnpaste einen Zusammenhang in Form einer Hysteresekurve, wenn die Schergeschwindigkeit zyklisch zwischen 0 und einem bestimmten Wert verändert wird, so beziehen sich die im Rahmen dieser Anmeldung genannten Zahlenwerte für Fließgrenze und Viskosität in allen Fällen auf den sog. Aufwärtsschergang. Dies ist der Meßabschnitt, bei dem die Schergeschwindigkeit von 0 auf den Endwert erhöht wird. Das verwendete Meß- und Auswerte-Verfahren ist ausführlich im Beispiel-Teil dargestellt.

Als besonders geeignet für den erfindungsgemäßen Einsatz haben sich Zahnpasten erwiesen, bei denen das Verhältnis der Fließgrenzen von Trägerstrangmasse und Streifenmasse 1,12:1 bis 1,18:1, insbesondere 1,15:1, beträgt. Bei Raumtemperatur liegen die Absolutwerte der Fließgrenzen für die erfindungsgemäßen Zahnpasten im Bereich von etwa 65 - 280 Pa.

Weiterhin ist es vorteilhaft, wenn sich die übrigen rheologischen Eigenschaften von Trägerstrangmasse und Streifenmasse nur wenig unterscheiden. So ist es vor allem günstig, wenn die dynamischen Viskositäten, die bei Schubspannungen oberhalb der Fließgrenzen gemessen werden, möglichst geringe Unterschiede aufweisen. Diese dynamischen Viskositäten liegen für die erfindungsgemäßen Zahnpasten bei Raumtemperatur in einem Bereich von etwa 0,01 bis 0,05 Pas.

Dies kann beispielsweise dadurch erreicht werden, daß sich Trägerstrangmasse und Streifenmasse nur durch den Gehalt an Wasser und/oder anderen zur Einstellung der Fließgrenzen dienenden Zusatzstoffen sowie Farbstoffen und/oder Pigmenten unterscheiden. So können Trägerstrangmasse und Streifenmasse auf Basis einer Grundmischung formuliert werden. Dieser Grundmischung werden dann die genannten Komponenten zugemischt.

Die erfindungsgemäßen Zahnpasten können in Form von gelförmigen oder pastösen Zubereitungen aus den bekannten Komponenten hergestellt werden. Übliche Komponenten sind Schleif- und Putzmittel, Wasser, Feuchthaltemittel, Verdickungsmittel, Tenside, Aromastoffe, Süßungsmittel, Antiseptika und mundkosmetisch wirkende Stoffe sowie Farbstoffe.

Als Schleif- und Putzmittel eignen sich beispielsweise spezielle Kieselsäuren wie Fällungskieselsäuren und Gelkieselsäuren, Aluminiumoxid-trihydrat, feinteiliges $\alpha$-Aluminiumoxid, Calciumcarbonat, Calciumphosphat, Magnesiumphosphat, wasserunlösliches Natriummetaphosphat, Magnesiumcarbonat und Magnesiumhydroxid sowie Mischungen dieser Verbindungen. Die Schleif- und Putzmittel werden üblicherweise in Mengen von etwa 15-45 Gew.-%, bezogen auf die Gesamtmasse der Zahnpaste, eingesetzt.

Als Feuchthaltemittel dienende Substanzen sind beispielsweise Polyalkohole wie z.B. Glycerin, Sorbit und Xylit.

Zum Verdicken der Zubereitungen können zum Beispiel Aerogelkieselsäuren, Bentonite und Hydrokolloide wie Carboxymethylcellulose, Hydroxyethylcellulose, Hydroxypropylguar, Hydroxyethylstärke, Polyvinylpyrrolidon, Polyethylenglykole, Pflanzengumme wie Traganth, Agar-Agar, Carragheenane, Gummi arabicum und Xanthane sowie Carboxyvinylpolymere wie die bekannten Carbopol[(R)]-Typen verwendet werden.

Weiterhin können oberflächenaktive Stoffe, bevorzugt anionische schaumstarke Tenside, wie z.B. lineare Natriumalkylsulfate mit 12-18 C-Atomen in der Alkylgruppe, Natriumsalze von Alkylpolyglykolethersulfaten mit 12-16 C-Atomen in der linearen Alkylgruppe und 2-6 Glykolethergruppen im Molekül, von linearen Alkan-($C_{12}$-$C_{18}$)-sulfonaten, Sulfobernsteinsäuremonoalkyl-($C_{12}$-$C_{18}$)-estern, sulfatierten Fettsäuremonoglyceriden, sulfatierten Fettsäurealkanolamiden, Sulfoessigsäurealkyl-($C_{12}$-$C_{18}$)-estern, Acylsarkosiden, Acyltauriden und Acylisethionaten mit jeweils 8-18 C-Atomen in der Acylgruppe enthalten sein. Auch nichtionische Tenside sind geeignet, z.B. Oxethylate von Fettsäuremono- und -diglyceriden, Fettsäuresorbitanestern und Ethylenoxid-Propylenoxid-Blockpolymerisate.

Als Aromastoffe eignen sich beispielsweise Pfefferminzöl, Krauseminzöl, Eukalyptusöl, Anisöl, Fenchelöl, Kümmelöl, Menthylacetat, Zimtaldehyd, Anethol, Vanillin und Thymol sowie Mischungen dieser und anderer natürlicher und synthetischer Aromen. Es kann bevorzugt sein, Aromastoffe zu verwenden, die der Zahnpaste einen Geschmack verleihen, der dem von Früchten wie Erdbeere, Himbeere, Ananas, Apfel oder Orange entspricht.

Als Süßungsmittel können die Zahnpasten Substanzen wie Saccharin, Natriumcyclamat, Aspartyl-phenylalanin-methylester und Acesulfam[R]Ksowie Sucrose, Lactose, Maltose und Fructose enthalten.

Unter Antiseptika und mundkosmetisch wirkenden Stoffe sind Konservierungsmittel und antimikrobielle Stoffe, Antizahnsteinwirkstoffe, karieshemmende, Plaque-inhibierende Stoffe sowie entzündungshemmende Substanzen zu verstehen.

Geeignete Konservierungsmittel und antimikrobielle Stoffe sind u.a. p-Hydroxybenzoesäuremethyl-, -ethyl- und -propylester, Natriumsorbat, Natriumbenzoat, Bromchlorophen, Phenylsalicylsäureester und Thymol.

Als Antizahnsteinwirkstoffe können Azacycloheptan-2,2-diphosphonsäure, 1-Hydroxyethan-1,1-diphosphonsäure, 1-Phosphonopropan-1,2,3-tricarbonsäure und deren Natriumsalze sowie die aus US-PS 3,488,419, DE-OS 22 24 430 und DE-OS 23 43 196 bekannten Verbindungen verwendet werden.

Karieshemmende Stoffe sind beispielsweise Natriumfluorid, Natriummonofluorphosphat und Zinnfluorid.

Geeignete keratogene oder entzündungshemmende Stoffe sind z.B. Allantoin, Harnstoff, Azulen, Kamillewirkstoffe und Acetylsalicylsäurederivate.

Schließlich können die erfindungsgemäßen Zahnpasten noch Farbstoffe und/oder Pigmente wie z.B. Titandioxid sowie Puffersubstanzen wie beispielsweise primäre, sekundäre oder tertiäre Alkaliphosphate oder Citronensäure/Natriumcitrat enthalten.

Die zur Einstellung der Fließgrenzen bevorzugt dienenden Zusatzstoffe sind Polyalkohole, insbesondere Sorbit und/oder Glycerin, und/oder Kieselsäure.

Erfindungsgemäße Zahnpasten zeigen dann ein besonders vorteilhaftes ästhetisches Aussehen, wenn sie Trägerstrangmasse und Streifenmasse in einem Volumenverhältnis von etwa 92:8 bis etwa 97:3, insbesondere von 95:5, enthalten.

Im Rahmen der erfindungsgemäßen Lehre können sowohl Trägerstrangmasse als auch Streifenmasse unabhängig voneinander so formuliert werden, daß sie transparent oder intransparent erscheinen. Die entsprechenden Maßnahmen, beispielsweise die Zugabe von Pigmenten für intransparente Formulierungen, sind dem Fachmann bekannt.

Es ist weiterhin besonders bevorzugt, daß die Zahnpaste aus dem Vorratsbehälter als Strang mit einem Querschnitt in Form eines n-Eckes, wobei n eine ganze Zahl von 3 bis 7 ist, aus dem Vorratsbehälter abgegeben wird und sich die Streifenmasse in den Ecken des n-Eckes befindet. Ganz besonders bevorzugt ist die Abgabe in Form eines Stranges mit einem Querschnitt in Form eines Rechteckes, insbesondere in Form eines Quadrates.

Die Teilaufgabe bezüglich der Vorrichtung wird bei einer gattungsgemäßen Vorrichtung dadurch gelöst, daß die Austrittsöffnung im Bereich des Halses eine mehreckige, insbesondere quadratische, Querschnittsfläche aufweist und daß die Durchbrechungen in den Ecken der Querschnittsfläche angeordnet sind.

Hierdurch wird ein Austragsnippel für Zahnpastenbehälter geschaffen, mit dem ein mehreckiger Zahnpastenstrang aus dem Behälter gefördert werden kann, bei welchem die Streifen die Kanten bilden.

Nachstehend ist eine Vorrichtung für die Verwendung zum Ausbringen der erfindungsgemäßen Zahnpaste beispielsweise näher erläutert. Diese zeigt in

Figur 1 einen Längsschnitt durch die Vorrichtung und in

Figur 2 eine Aufsicht.

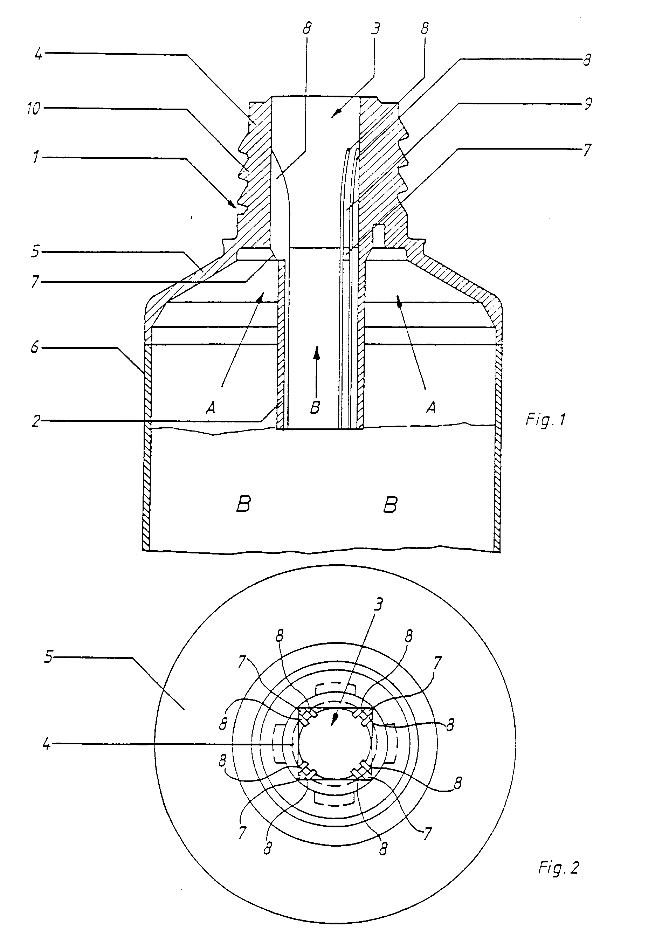

Die Vorrichtung besteht aus einem insgesamt mit (1) bezeichneten Austragsnippel, der ein Steigrohr (2) aufweist, welches in einen, die Austragsöffnung (3) umfassenden Hals (4) übergeht. An den Hals (4) ist ringförmig eine Schulter (5) angeformt, die anschließend in eine Behälterwandung (6) übergeht. Beispielsweise ist die Behälterwandung (6) die Wand einer Laminattube. Insoweit ist in der Figur 1 der obere Bereich einer Tube dargestellt, wie er von üblichen Zahnpastentuben zur Abgabe von Streifenzahncreme bekannt ist. Im Unterschied zu diesen üblichen Tuben weist nun der Nippel (1) im Bereich des Übergangs vom Steigrohr (2) zum Hals (4), der in dem Bereich erfolgt, in welchem auch die Schulter (5) an den Hals (4) angeformt ist, eine Querschnittserweiterung und -veränderung auf. Während das Steigrohr (2) einen rundzylinderförmigen Querschnitt aufweist und somit durch das Steigrohr (2) ein runder Strang eines Produktes B zu fördern ist, erweitert sich der Querschnitt der Austrittsöffnung (3) im Bereich des Halses (4) zu einem quadratischen Querschnitt. Im Übergangsbereich von dem konzentrisch zu der Austrittsöffnung (3) angeordneten Steigrohr (2) zu dem Hals (4) sind am Innenumfang verteilt vier Durchbrechungen (7) ausgebildet. Diese Durchbrechungen (7) liegen genau in den vier Ecken der quadratischen, von dem Hals (4) umfaßten, Austrittsöffnung (3). An den Längsrändern der Durchbrechungen (7) vorbeiführend sind über die Länge des Steigrohres (2), bis kurz unter den oberen Rand des Halses (4) reichend, schmale Stege (8) ausgebildet, die geringfügig aus der Innenoberfläche von Steigrohr (2) und Hals (4) hervorstehen und schmale Kanäle (9) bilden. In diesen Kanälen (9) wird oberhalb der Durchbrechungen (7) eine Streifenmasse A geführt.

In dem von den Behälterwandungen (6) umfaßten Behälter ist üblicherweise bis zum unteren Rand des Steigrohres (2) eine Trägerstrangmasse B und in dem verbleibenden Ringraum zwischen Außenoberfläche des Steigrohres (2) und der Behälterwandung (6) sowie unterhalb der Schulter (5) befindet sich üblicherweise die Streifenmasse A. Insgesamt ist ein derartiger Behälter üblicherweise zu 95 % mit der Trägerstrangmasse B und zu 5 % mit der Streifenmasse A gefüllt.

Wird nun ein Druck auf die Trägerstrangmasse B ausgeübt, bei einer flexiblen Behälterwandung (6) üblicherweise durch Fingerdruck von außen, so überträgt die Trägermasse B diesen Druck auf die Streifenmasse A und es tritt durch das Steigrohr (2) die Trägerstrangmasse B und durch die Durchbrechungen (7) die Streifenmasse A in die Behälteröffnung (3) ein. Aufgrund der Ausbildung des Halses (4) bildet sich in dessen Bereich ein quadratischer Zahncremestrang, dessen Eckkanten von der Streifenmasse A gebildet sind. Bei unterschiedlicher Farbgestaltung von Trägerstrangmasse B und Streifenmasse A erhält man dann einen Zahnpastenstrang mit farblich abgesetzten Streifen auf den Eckkanten.

Auf der Außenfläche des Halses (4) ist ein Gewinde (10) aufgebildet, auf welches ein üblicher Verschluß zum Verschließen der Austrittsöffnung (3) aufgeschraubt werden kann.

**Beispiele**:

Die nachfolgenden Rezepturbeispiele dienen zur weiteren Erläuterung der Erfindung.

## 1. Zahnpasten-Grundmasse

Zur Formulierung einer erfindungsgemäßen Zahnpaste wird zunächst die im folgenden angegebene transparente Grundmischung hergestellt:

| Komponente | Gewichtsteile |
|---|---|
| Fällungskieselsäure SIDENT[R] 12 DS[1] | 19,00 |
| Aerogelkieselsäure FK 300 DS[2] | 2,00 |
| Sorbit (70%ige Lösung in Wasser) | 45,00 |
| Glycerin (86%ige Lösung in Wasser) | 18,00 |
| Wasser | 7,77 |
| Polyethylenglykol 300[3] | 2,00 |
| Blanose refined CMC 7M2[4] | 0,40 |
| Natriumlaurylsulfat | 1,50 |
| Natriumfluorid | 0,23 |
| Hydroxybenzoesäuremethylester | 0,10 |
| Saccharin | 0,30 |
| Aromastoffe | 0,90 |

1 (DEGUSSA)
2 (DEGUSSA)
3 (HÜLS)
4 Carboxymethylcellulose-Natrium-Salz (AQUALON)

Auf Basis dieser Grundmischung wurden folgende Zahnpasten formuliert (die Mengenangaben sind jeweils Gewichtsteile):

| 1. Zahnpaste mit opaker Trägerstrangmasse und intransparenter Streifenmasse | | |
|---|---|---|
| | Trägerstrangmasse | Streifenmasse |
| Grundmischung | 97,2 | 97,2 |
| Sorbit (70% in Wasser) oder Glycerin (86% in Wasser) | - | 2,0 |
| Wasser (voll entsalzt) | 2,1 | - |
| Titandioxid | - | 0,8 |
| Aerogelkieselsäure FK 300 DS | 0,7 | - |
| Farbstoffe | < 0,05 | < 0,05 |
| Fließgrenze (Pa) bei 20 °C | 257 | 222 |
| Viskosität (Pas) bei 20 °C | 0,02 | 0,02 |

| 2. Zahnpaste mit transparenter Trägerstrang- und Streifenmasse | | |
|---|---|---|
| | Trägerstrangmasse | Streifenmasse |
| Grundmischung | 98,0 | 98,0 |
| Sorbit (70% in Wasser) oder Glycerin (86% in Wasser) | - | 2,0 |
| Wasser (voll entsalzt) | 1,3 | - |
| Aerogelkieselsäure FK 300 DS | 0,7 | - |
| Farbstoffe | < 0,05 | < 0,05 |
| Fließgrenze (Pa) bei 20 °C | 227 | 200 |
| Viskosität (Pas) bei 20 °C | 0,02 | 0,03 |

Die Viskositätsmessungen wurden mit dem Meßsystem Rotovisco[R] RV 12 der Fa. Haake unter Verwendung des Meßeinrichtung SV II St und des Meßkopfes 150 vorgenommen. Zur Steuerung des Antriebes des Meßkopfes diente der Haake Rheocontroller[R].

Der Meßvorgang wurde bei einer Temperatur von 20 °C durchgeführt. Dazu wurde zunächst die Schergeschwindigkeit im Verlauf von 180 Sekunden kontinuierlich von D = 0 auf 128 min$^{-1}$ erhöht (Aufwärtsschergang). Nach einer Haltezeit von 6 Sekunden bei D = 128 min$^{-1}$ wurde die Schwergeschwindigkeit im Verlauf von 180 Sekunden kontinuierlich von 128 auf 0 min$^{-1}$ erniedrigt.

Trägt man den ermittelten Zusammenhang zwischen Schwergeschwindigkeit und Schubspannung graphisch auf, so wurde in der Regel eine Hysteresekurve erhalten.

Die Ausgangssignale des Rotovisco[R] RV 12 wurden über ein Interface auf einen Rechner übertragen, mit dessen Hilfe die Auswertung vorgenommen wurde. Dazu wurde aus den Meßwerten für die Schubspannung bei D = 32 min$^{-1}$ und D = 128 min$^{-1}$ durch eine Regressonsrechnung auf Basis der Casson-Gleichung die Fließgrenze bei D = 0 berechnet.

Die Zahlenwerte für die Fließgrenzen und die Viskositäten in den obigen Tabellen beziehen sich jeweils auf den Aufwärtsschergang. Die angegebenen Zahlenwerte sind Werte, die die Zahnpastenmassen nach einer Lagerung von etwa einer Woche erreichen und die sich bei weiterer Lagerung im Rahmen der Meßgenauigkeit nicht mehr verändern. Üblicherweise werden direkt nach Zubereitung der Zahnpastenmassen deutlich niedrigere Werte für die Fließgrenze und deutlich höhere Werte für die Viskosität gemessen. Diese Werte verändern sich in den erste Stunden und Tagen zunächst stark und erreichen nach etwa einer Woche asymptotisch die angegebenen Endwerte.

**Patentansprüche**

1. Zahnpaste zum Ausbringen aus einem Behälter in Form eines mehrfarbigen, gestreiften Stranges bestehend aus einer Trägerstrangmasse sowie mindestens einer, in der Farbe von der Trägerstrangmasse verschiedenen Streifenmasse, dadurch gekennzeichnet, daß das Verhältnis der Fließgrenzen von Trägerstrangmasse und Streifenmasse 1,10:1 bis 1,20:1 beträgt.

2. Zahnpaste nach Anspruch 1, dadurch gekennzeichnet, daß das Verhältnis der Fließgrenzen 1,12:1 bis 1,18:1, insbesondere 1,15:1, beträgt.

3. Zahnpaste nach einem der Ansprüche 1 oder 2, dadurch gekennzeichnet, daß sich Trägerstrangmasse und Streifenmasse nur durch den Gehalt an Wasser, anderer zur Einstellung der Fließgrenzen dienenden Zusatzstoffen sowie Farbstoffen und/oder Pigmenten unterscheiden.

4. Zahnpaste nach Anspruch 3, dadurch gekennzeichnet, daß die zur Einstellung der Fließgrenzen dienenden Zusatzstoffe Polyalkohole, insbesondere Sorbit und/oder Glycerin, und/oder Kieselsäure sind.

5. Zahnpaste nach einem der Ansprüche 1 bis 4, dadurch gekennzeichnet, daß sie Trägerstrangmasse und Streifenmasse in einem Volumenverhältnis von 92:8 bis 97:3, insbesondere von 95:5, enthält.

6. Zahnpaste nach einem der Ansprüche 1 bis 5, dadurch gekennzeichnet, daß sie als Strang mit einem Querschnitt in Form eines n-Eckes, wobei n eine ganze Zahl von 3 bis 7 ist, aus dem Vorratsbehälter abgegeben wird und sich die Streifenmasse in den Ecken des n-Eckes befindet.

7. Zahnpaste nach Anspruch 5, dadurch gekennzeichnet, daß sie als Strang mit einem Querschnitt in Form eines Rechteckes, insbesondere in Form eines Quadrates, aus dem Vorratsbehälter abgegeben wird.

8. Vorrichtung zur Verwendung für das Ausbringen von streifenförmiger Zahnpaste, insbesondere von Zahnpaste nach einem der Ansprüche 6 oder 7, aus einem Behälter, wie beispielsweise einer Tube oder einem Spender, bestehend aus einem Nippel (1) mit einem Hals (4) mit Austrittsöffnung (3) und angeformter Ringschulter (5), wobei innerhalb des Nippels (1) unterhalb des Ansatzes der Ringschulter (5) an den Hals (4) konzentrisch zur Austrittsöffnung (3) ein rundzylinderförmiges Steigrohr (2) ausgebildet ist, welches in seinem Übergangsbereich zu dem Hals (4) Durchbrechungen (7) aufweist, dadurch gekennzeichnet, daß die Austrittsöffnung (3) im Bereich des Halses (4) eine mehreckige, insbesondere quadratische Querschnittsfläche aufweist, und daß die Durchbrechungen (7) in den Ecken der Querschnittsfläche angeordnet sind.

**Claims**

1. A toothpaste to be dispensed from a container in the form of a multicoloured, striped strand consisting of a carrier strand paste and at least one stripe paste differing in colour from the carrier strand paste, characterized in that the ratio between the yield points of the carrier strand paste and the stripe paste is 1.10:1 to 1.20:1.

2. A toothpaste as claimed in claim 1, characterized in that the ratio between the yield points is 1.12:1 to 1.18:1 and more especially 1.15:1.

3. A toothpaste as claimed in claim 1 or 2, characterized in that the carrier strand paste and the stripe paste differ solely in their contents of water, other additives used for establishing the yield points and also dyes and/or pigments.

4. A toothpaste as claimed in claim 3, characterized in that the additives used to establish the yield points are polyalcohols, particularly sorbitol and glycerol, and/or silica.

5. A toothpaste as claimed in any of claims 1 to 4, characterized in that it contains the carrier strand paste and the stripe paste in a ratio by volume of 92:8 to 97:3 and more especially 95:5.

6. A toothpaste as claimed in any of claims 1 to 5, characterized in that it is dispensed from a storage container as a strand with a cross-section in the form of an n-angle, where n is an integer of 3 to 7, the stripe paste being situated at the corners of the n-angle.

7. A toothpaste as claimed in claim 5, characterized in that it is dispensed from the storage container as a strand with a cross-section in the form of a rectangle and more particularly in the form of a square.

EP 0 373 469 B1

8. A device for dispensing striped toothpaste, more especially the toothpaste claimed in claim 6 or 7, from a container, such as for example a tube or a dispenser, consisting of a nipple (1) with a neck (4) having an outlet opening (3) and surrounded by an annular shoulder (5), a cylindrical riser tube (2) with interruptions (7) in its transition zone to the neck (4) being arranged concentrically to the outlet opening (3) inside the nipple (1) below the shoulder (5) surrounding the neck (4), characterized in that the outlet opening (3) of the neck (4) has a polygonal and more especially square cross-sectional area and in that the interruptions (7) are arranged at the corners of the cross-sectional area.

**Revendications**

1. Pâte dentifrice pour être distribuée à partir d'un réservoir sous forme d'un cordon à raies, ainsi que d'au moins une masse à raies différentes dans la couleur de la masse de cordon de support, caractérisée en ce que le rapport des limites d'écoulement de la masse de cordon de support et de la masse à raies s'élève de 1,10:1 à 1,20:1.

2. Pâte dentifrice selon la revendication 1, caractérisée en ce que le rapport des limites d'écoulement s'élève de 1,12:1 à 1,18:1 en particulier à 1,15:1.

3. Pâte dentifrice selon l'une des revendications 1 ou 2, caractérisée en ce que la masse de cordon de support et la masse à raies ne se différencient que par la teneur en eau, par d'autres substances additionnelles qui servent à l'ajustement des limites d'écoulement ainsi que par les colorants et/ou les pigments.

4. Pâte dentifrice selon la revendication 3, caractérisée en ce que les substances additionnelles qui servent à l'ajustement des limites d'écoulement sont des polyalcools, en particulier du sorbitol et/ou du glycérol et/ou de l'acide silicique.

5. Pâte dentifrice selon l'une des revendications 1 à 4, caractérisée en ce qu'elle renferme une masse de cordon de support et une masse à raies dans un rapport volumique de 92:8 à 97:3, en particulier de 95:5.

6. Pâte dentifrice selon l'une des revendications 1 à 5, caractérisée en ce qu'elle est extraite sous forme de cordon ayant une section transversale en forme d'un polygone à n côtés dans lequel n est un nombre entier de 3 à 7, du réservoir d'alimentation et que la masse à raies se trouve dans les angles du polygone n.

7. Pâte dentifrice selon la revendication 5, caractérisée en ce qu'elle est extraite sous forme de cordon ayant une section transversale en forme de rectangle - en particulier en forme d'un carré - du réservoir d'alimentation.

8. Dispositif pour l'utilisation pour distribution des pâtes dentifrices sous forme de raies, en particulier de pâtes dentifrices selon l'une des revendications 6 ou 7, d'un réservoir comme par exemple un tube ou un distributeur, qui se compose d'un raccord (1) avec un goulot (4) ayant un orifice de sortie (3) et un épaulement circulaire (5) adapté, dans lequel à l'intérieur du raccord (1) en dessous de la pièce de raccordement de l'épaulement circulaire (5) sur le goulot (4) un tube montant (2) en forme de cylindre rond est réalisé concentriquement à l'orifice de sortie (3), tube qui possède dans sa zone de transition vers le goulot (4), des découpes (7), caractérisé en ce que l'orifice de sortie (3) possède dans la zone du goulot (4) une surface de section transversale polygonale, en particulier quadratique, et que les découpes (7) sont disposées dans les angles de la surface de section transversale.

8

Fig. 1

Fig. 2